# EUROPÄISCHE PATENTSCHRIFT

(11) **EP 0 617 922 B1**
(45) Veröffentlichungstag und Bekanntmachung des Hinweises auf die Patenterteilung: **17.12.1997**
(21) Anmeldenummer: 94810130.8
(22) Anmeldetag: 02.03.1994
(51) Int. Cl.: A61B 8/08, A61B 8/00

(54) **Ultraschall-Doppler-Sonde mit Nadelführung**
Doppler ultrasound probe with needle guide
Sonde d'ultrasons à effet Doppler avec un guide d'aiguille

(30) Priorität: 01.04.1993 CH 1003/93
(43) Veröffentlichungstag der Anmeldung: 05.10.1994
(73) Patentinhaber: Bollinger, Armin, CH-8610 Uster (CH)
(72) Erfinder: Bollinger, Armin, CH-8610 Uster (CH)
(74) Vertreter: Feldmann, Clarence Paul

(56) Entgegenhaltungen:
- EP-A- 0 540 461
- CH-A- 501 410
- CH-A- 676 787
- DE-A- 3 244 667
- GB-A- 2 009 563
- US-A- 3 721 227
- US-A- 4 108 165

## Beschreibung

Die Erfindung betrifft eine Ultraschall-Doppler-Sonde mit Nadelführung, zum Orten von Blutgefässen in einem Körper und deren Anstechen mit einer Hohlnadel, mit einem auf dem Dopplerprinzip ausgelegten Ultraschallsender und Empfänger nach dem Oberbegriff des unabhängigen Patentanspruches.

Beim Behandeln im Operationssaal, Katheterlabor und bei verschiedenen anderen Eingriffen müssen, beispielsweise zum Einführen von Kathetern, Arterien oder Venen mit relativ dicken Hohlnadeln angestochen werden. Oft werden Arterien oder Venen punktiert, welche durch die Haut nicht gut sichtbar und kaum palpierbar sind. Es stellt sich daher das Problem, geeignete Blutgefässe im Körper überhaupt genügend genau zu lokalisieren, um diese dann anschliessend anstechen zu können. Die bekannte Ultraschall-Doppler Technik erlaubt ein einfaches und genaues Lokalisieren der gesuchten Blutgefässe. Dabei kann sogar die Strömungsrichtung des Blutes im einzelnen Blutgefäss und somit Arterien und Venen unterschieden und bestimmt werden.

Aus dem Patent CH 536'635 (Siemens) ist eine Vorrichtung bekannt, bei welcher eine Ultraschall-Doppler Sonde fest am oberen Ende einer Injektionskanüle angeordnet ist. Die Sonde kann nach der Punktion nicht von der Kanüle abgenommen werden.

Die Kanüle ist daher mit einer Abzweigung seitwärts zum Anschluss einer Spritze oder einer Leitung versehen. Mit dieser Anordnung können Injektionskanülen treffsicher in Blutgefässe eingeführt werden.

Diese Technik hat sich in der Praxis nicht durchgesetzt.

Ferner ist aus DE-A-3 244 667 eine Ultraschall-Sonde gemäß Oberbegriff des Anspruchs 1 bekannt.

Aus US 4'108'165 (Kopp) ist eine Ultraschall-Sonde mit einer konzentrisch längs in der Sonde angeordneten Nadelführung bekannt. Die Nadelfühung bildes das innere Ende eines Schlitzes, selcher von radial auss bis in konzentrischen Nadelführung reicht. Die Sonde kann dadurch von einer gesetzten Nadel seitlich weggezogen werden.

Aus CH 676 787 (Sulzer) ist ein Punktiergerät mit einer Ultraschall-Sonde bekannt. Der Sondenkopf weist ebenfalls einen Schlitz auf, durch welchen die Nadel geführt werden kann. Allerdings steht die Nadel immer in einem Winkel zum Sondenkopf. Dieses Gerät eignet sich daher zum Suchen und Punktieren von oberflächennahen Blutgefässen. Die Ultraschall-Sonde kann gegenüber einer gesetzten Nadel verschwenkt werden, um eine Weiterarbeiten weniger zu behindern.

Aufgabe der Erfindung ist, eine Ultraschall-Doppler-Sonde zu schaffen, mit welcher eine Hohlnadel zusammen benützbar ist. Die Hohlnadel soll gesetzt werden können nachdem das gesuchte Blutgefäss mit der Ultraschall-Doppler-Sonde geortet ist. Darauf soll die Ultraschall-Doppler-Sonde von der Hohlnadel getrennt und entfernt werden können, während die Hohlnadel am Ort gesetzt verbleibt.

Diese Aufgabe wird durch die im Anspruch 1 angegebene Erfindung gelöst.

Ein massgebender Vorteil der Erfindung ist die Möglichkeit, die Ultraschall-Sonde von der Nadel seitwärts zu entfernen, nachdem das gesuchte Blutgefäss punktiert ist. Dadurch befinden sich keine unnötigen behindernden Leitungen mehr im Arbeitsbereich des Arztes.

Ein zusätzlicher Vorteil ist, dass die Ultraschall-Doppler-Sonde zusätzlich zur Ortung des Blutgefässes auch die Strömungsrichtung darin eindeutig angibt. Dadurch kann beispielsweise eindeutig zwischen Arterien und Venen unterschieden werden. Bei Verwendung eines bidirektionalen Dopplergerätes kann die Strömungsrichtung optisch an der Sonde selbst angezeigt werden. Ein unidirektionales Dopplergerät gibt die Signale akustisch an.

Die erfindungsgemässe Ultraschall-Doppler-Sonde wird nachstehend im Zusammenhang mit den Zeichnungen beschrieben. Dabei zeigen:
Figur 1 eine Variante des teilbaren Sondenkopfes mit seitlich ausfahrbarer Hohlnadel in Aufsicht;
Figur 2 die gleiche Variante mit Hohlnadel in Seitenansicht
Figur 3 eine Variante mit teilbarem stufenförmigen Sondenkopf in Vorderansicht;

Eine bekannte Ultraschall-Doppler-Sonde mit Ultraschallsender und Empfänger arbeitet über eine elektrische Leitung mit einem unidirektionalen oder bidirektionalen Dopplergerät zur Signalauswertung zusammen. Der Sender in der Sonde kann im kontinuierlichen oder im gepulsten Verfahren betrieben werden. Das Dopplergerät enthält einen Verstärker und andere notwendige bekannte elektronische Elemente. Die Anzeige erfolgt entweder akustisch durch einen Lautsprecher oder optisch, beispielsweise mittels einer LED oder LCD Anzeige.

Erfindungsgemäss wird in einer einfachsten Variante eine Ultraschall-Doppler-Sonde so ausgeführt, dass in ihr der Sondenkopf in einer einteiligen Halterung zweiteilig ausgeführt wird. Im einen Teil ist der Piezokristall zum Senden und im anderen Teil der Piezokristall zum Empfangen angeordnet. Die beiden Teile des Sondenkopfes liegen an einer Trennebene so dicht aneinander an, dass die Funktion der Sonde gewährleistet ist. Vom Rand des Sondenkopfes ist ein schmaler Schlitz entlang der Trennebene radial einwärts bis ins Zentrum des Sondenkopfes eingelassen. Dieser Schlitz bildet im Zentrum eine Nadelführung von oben nach unten durch den Sondenkopf. Nach dem Orten des Blutgefässes wird eine Hohlnadel entlang dieser Nadelführung ins Blutgefäss gesetzt. Anschliessend kann die Ultraschall-Doppler-Sonde seitwärts von der gesetzten Hohlnadel entlang dem Schlitz weggezogen werden.

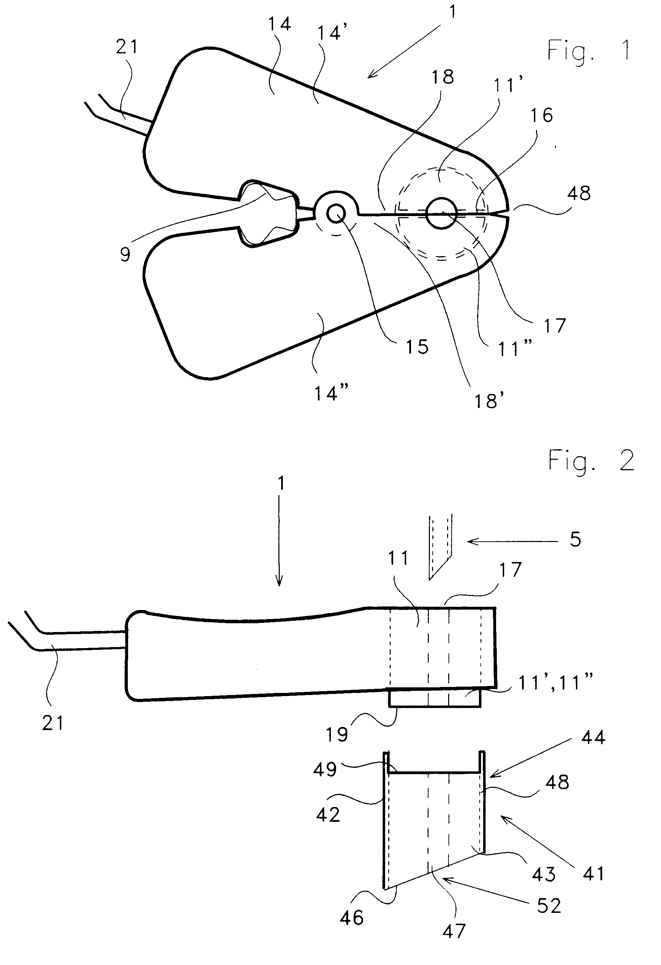

Eine optimierte Variante der Ultraschall-Doppler-Sonde ist in Figur 1 von oben gesehen dargestellt. Sie besteht aus einer Halterung 14 mit einem Sondenkopf 11. Die Halterung 14 ist im Prinzip eine Federklemme. Die Halterung 14 umfasst zwei Klemmenhälften 14', 14'', welche etwa in der Mitte mit einem Schwenkgelenk 15 miteinander schwenkbar verbunden sind und mittels einer Zuhaltefeder in einem Ruhezustand gehalten werden. Die beiden Klemmenhälften 14', 14'' sind auf einer Seite des Schwenkgelenkes 15 mit Klemmwangen 18,18' versehen. Diese Klemmwangen 18,18' liegen im Ruhezustand satt aneinander an. Auf der gegenüberliegenden Seite des Schwenkgelenkes 15 weisen die beiden Klemmenhälften 14', 14'' in einem Winkel auseinander. Dadurch kann die Halterung hier gegen die Kraft einer Zuhaltefeder 19 zusammengedrückt werden, wodurch sich die Halterung 14 im Bereich der Klemmwangen 18,18' öffnet.

Der Sondenkopf 11 mit Ultraschall-Sender 11' und -Empfänger 11'' befindet sich in der Halterung 14 im Bereich der aneinander anliegenden Klemmwangen 18, 18'. Eine Trennebene 16 ist durch die Klemmwangen 18, 18' beider Klemmenhälften gebildet. Die Trennebene 16 führt daher auch durch den Sondenkopf 11, so dass dieser auch zweigeteilt ist. Der Sender 11' ist in der Klemmwange 18 der einen Klemmenhälfte 14' integriert. Der Empfänger 11'' ist in der Klemmwange 18' der anderen Klemmenhälfte 14'' integriert. Sender 11' und Empfänger 11'' liegen im Ruhe- und Arbeitszustand der Halterung 14 so dicht aneinander an, dass der Sondenkopf funktionsfähig ist. Das heisst, Sender 11' und Empfänger 11'' liegen im Normalzustand durch den Federdruck in der richtigen Position, um die Ortung der Blutgefässe zu ermöglichen.

Die Nadelführung 17 ist konzentrisch im Sondenkopf 11 angeordnet. Sie führt als Bohrung von oben nach unten quer durch den Sondenkopf 11. Auch diese Nadelführung 17 wird durch die Trennebene 16 in zwei Hälften geteilt.

Die Ultraschall-Doppler-Sonde 1 wird mit einer Hand gehalten und auf einem zu untersuchenden Körper bewegt. Die Signale der Sonde gelangen über die Kabel 21 zum Dopplergerät mit der bekannten akustischen und/oder optischen Anzeige. Sobald das gesuchte Blutgefäss geortet ist, wird die Hohlnadel 5 durch die Nadelführung 17 hindurch in den Körper gestossen, bis das Blutgefäss punktiert ist. Die konzentrische Anordnung der Nadelführung 17 im Sondenkopf 11 gewährleistet ein zuverlässiges Treffen des georteten Blutgefässes. Anschliessend wird die Halterung 14 an ihrem offenen Ende gegen die Kraft der Zuhaltefeder 9 zusammengedrückt. Dadurch wird die Halterung 14 im Bereich der Trennebene 16 der Klemmwangen 18, 18' gespreizt und öffnet sich keilförmig mitsamt dem Sondenkopf 11 und der Nadelführung 17. Die Hohlnadel 5 wird freigegeben und die Ultraschall-Doppler-Sonde 1 kann von der im Körper gesetzten Hohlnadel 5 seitlich weggezogen werden.

Die Halterung 14 ist ergonomisch so gestaltet, dass eine Person sie mit nur einer Hand halten, führen und in Position sichern kann und die zweite Hand für die Führung von Hohlnadel und Spritze frei bleibt.

In Figur 2 ist die Ultraschall-Doppler-Sonde 1 in Seitenansicht dargestellt.. Die Lage des Sondenkopfes 11 mit Sender 11', Empfänger 11'' und der Nadelführung 17 ist gut ersichtlich. Sender 11' und Empfänger 11'' ragen nach unten etwas aus der Halterung 14 heraus und bilden hier eine ebene Kontaktfläche 19. Die Kontaktfläche 19 dient der Uebertragung der Ultraschallwellen von der Sonde zu einem zu untersuchenden Körper. Die Nadelführung 17 steht rechtwinklig zur Kontaktfläche 19. Dadurch ist ein präzises Setzen der Nadel in das gesucht Blutgefäss gewährleistet.

Die Ultraschall-Doppler-Sonde 1 wird zusätzlich mit einem auswechselbaren Kopplungsteil 41 als Schallübertragungselement bestückt. Der Kopplungsteil 41 besteht aus einem zylindrischen Formteil 42 mit einer Füllung 43 aus geeignetem Material mit den notwendigen Ultraschall-Eigenschaften. Der Kopplungsteil 41 umfasst den Sondenkopf teilweise. Eine plane Oberfläche 49 stellt im Gebrauch den Kontakt zur Kontaktfläche 19 des Sondenkopfes 11 der Ultraschall-Doppler-Sonde 1 dar. Der Kopplungsteil 41 weist zum durchstossen der Hohlnadel 5 eine durchgehende Längsbohrung 47 auf. Von der Längsbohrung 47 erstreckt sich radial ein Schnitt als Trennfläche 48. Diese Trennfläche 48 entspricht der Trennebene 16 des Sondenkopfes und bildet deren Verlängerung im Kopplungsteil 41.

Der Sondenkopf 11 ragt in einen montierten Kopplungsteil 41 soweit nach unten, bis er mit seiner Kontaktfläche 19 mit der planen Oberfläche 49 der Füllung in Kontakt steht. Beim seitlichen Drücken auf die Halterung 14 öffnet sich der teilbare Sondenkopf 11. Dabei spreizt er auch den Formteil 42. Dieser öffnet sich an der Trennfläche schlitzförmig. Die Hohlnadel 5 wird freigegeben. Die Sonde 1 mit dem Kopplungsteil 41 kann seitlich ausgefahren werden.

Der Kopplungsteil 41 endet körperseitig in einer gegenüber der Längsachse der Nadelführung 17 geneigten Kontaktfläche 46. Es empfiehlt sich eine Neigung der Kontaktfläche 46 des Kopplungsteils 41 von etwa 45 Grad zu wählen. Die Handhabung kann durch eine geeignete Neigung dem Stechzweck entsprechend gewählt werden. Dadurch kann die Sonde im Gebrauch leicht schräg vom Körper weg gehalten werden und ist somit leichter handzuhaben. Um eine gute Ultraschalleitung zu gewährleisten, müssen die Kontaktflächen 46 und 19, sowie die Haut mit sterilem Wasser befeuchtet werden.

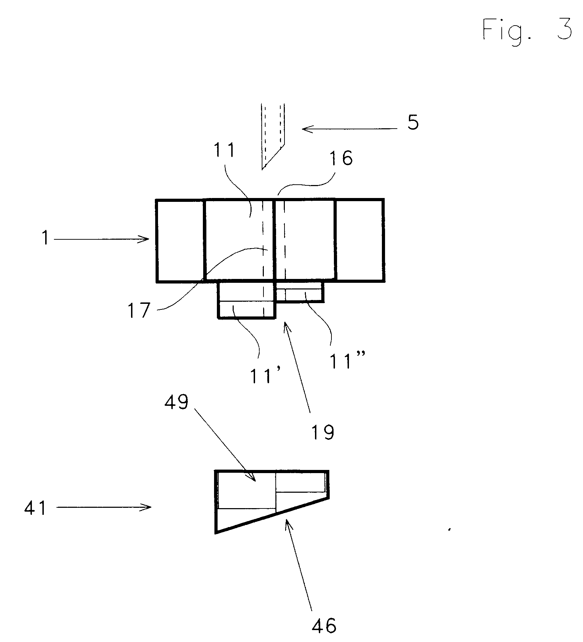

In Figur 3 ist die teilbare Sonde 1 von vorne gesehen dargestellt. Die Ultraschall-Doppler-Sonde 1 weist einen teilbaren Sondenkopf 11 auf, welcher nach unten verlängert ist. Dabei ist in dieser dargestellten Ausführungsform die Kontaktfläche 19 des Sondenkopfes 11 von vorne gesehen zweistufig ausgeführt. Der Sondenkopf 11 endet in einer ersten Stufe mit dem Sender 11' und in einer gegenüberliegenden zweiten Stufe mit dem Empfänger 11''. Die erste Stufe reicht weiter nach unten als die zweite Stufe. Es ist offensichtlich, dass diese Ausführungsform zusammen mit einem entsprechend ausgestalteten Kopplungsteil ausgerüstet werden muss. Der Kopplungsteil 41 weist dann eine gestufte Oberfläche 49 auf. Die zweistufige Ausführung hat den Vorteil, dass der Kopplungsteil 41 eine niedrige Bauhöhe bekommt, wodurch eine geringe Dämpfung erreicht werden kann.

## Patentansprüche

1. Ultraschall-Doppler-Sonde (1) zum Orten von Blutgefässen in einem Körper und deren Anstechen mit einer Hohlnadel (5), die Ultraschall-Doppler-Sonde (1) bestehend aus:
einem in einer Halterung (14) angeordneten Sondenkopf (11), welcher einen Ultraschallsender (11') und einen Ultraschallempfänger (11'') aufweist,
einer im Sondenkopf (11) als Bohrung angeordneten, vom Ultraschallsender (11') und Ultraschallempfänger (11'') mindestens teilweise umfassten Nadelführung (17), zum Einführen und Durchstossen der Hohlnadel (5), und einem auswechselbaren Kopplungsteil (41)
mit einer Füllung (43) aus Material mit annähernd gleichen Ultraschall-Eigenschaften wie der zu untersuchende Körper, wobei die Halterung (14) eine Federklemme mit zwei mittels einem Schwenkgelenk (15) verbundenen Klemmenhälften (14',14'') mit aneinander anliegenden, eine Trennebene (16) bestimmenden, Klemmwangen (18,18') ist, und wobei der Sondenkopf (11) in den Klemmwangen (18,18') so angeordnet ist, dass sich der Ultraschallsender 11' und der Ultraschallempfänger (11'') durch die Trennebene (16) geteilt in den beiden Klemmenhälften (14',14'') befinden,
dadurch gekennzeichnet, dass
der Sondenkopf (11) zylindrisch ausgebildet ist, dass die Bohrung koaxial zum Sondenkopf (11) angeordnet ist, dass
der Ultraschallsender (11') und der Ultraschallempfänger (11'') in Nadeleinstichrichtung aus der Halterung (14) herausragen und dass der Kopplungsteil (41) ein zylindrisches Formteil (42) ist, welches den Sondenkopf (11) teilweise umfasst, eine durchgehende Längsbohrung (47) zum Durchstossen der Hohlnadel (5) und eine Trennfläche (48), welche eine Verlängerung der Trennebene (16) bildet, aufweist.

2. Ultraschall-Doppler-Sonde nach Anspruch 1, dadurch gekennzeichnet, dass bei durch Zudrücken geöffneter Federklemme die Klemmwangen (18,18') gespreizt sind, so dass der Sondenkopf (11) und die Nadelführung (17) an der Trennebene (16) und der Kopplungsteil (41) an der Trennfläche (48) seitlich keilförmig geöffnet sind, wodurch die Ultraschall-Doppler-Sonde (1) mitsamt Kopplungsteil (41) seitwärts von der Hohlnadel (5) ausfahrbar ist.

3. Ultraschall-Doppler-Sonde nach Anspruch 1, dadurch gekennzeichnet, dass der auswechselbare Kopplungsteil (41) eine geneigte Kontaktfläche (46) zur Uebertragung der Schallwellen auf den zu untersuchenden Körper aufweist.

4. Ultraschall-Doppler-Sonde nach Anspruch 3, dadurch gekennzeichnet, dass der Sondenkopf (11) eine ebene Kontaktfläche (19) zur Uebertragung der Schallwellen über den Kopplungsteil (41) in einen zu untersuchenden Körper aufweist.

5. Ultraschall-Doppler-Sonde nach Anspruch 2, dadurch gekennzeichnet, dass der Sondenkopf (11) eine zweistufige Kontaktfläche (19) zur Uebertragung der Schallwellen über den Kopplungsteil (41) in einen zu untersuchenden Körper aufweist.

## Claims

1. An ultrasonic Doppler probe (1) for locating blood vessels in a body and their opening with a hollow needle (5), said ultrasonic Doppler probe (1) comprising a probe head (11) located in a support (14) and having an ultrasonic transmitter (11') and an ultrasonic receiver (11''), a needle guide (17) arranged as a bore in the probe head (11) and at least partly surrounded by the ultrasonic transmitter (11') and ultrasonic receiver (11''), for introducing and pushing through the hollow needle (5), and a replaceable coupling part (41) with a filling (43) of a material having approximately the same ultrasonic characteristics as the body to be examined, the support (14) being a spring clip with two clip halves (14', 14'') joined by means of a swivel hinge (15) and with clip cheeks (18, 18') abutting on one another and defining a separating plane (16), and in which the probe head (11) is so positioned in the clip cheeks (18, 18') that the ultrasonic transmitter (11') and the ultrasonic receiver (11'') are located in the two clip halves (14', 14''), divided by the separating plane (16), characterised in that the probe head (11) is constructed cylindrically, that the bore is positioned coaxially to the probe head (11), that the ultrasonic transmitter (11') and ultrasonic receiver (11'') project in the needle piercing direction out of the support (14) and that the coupling part (41) is a cylindrical moulding (42), which partly surrounds the probe head (11), has a through longitudinal bore (47) for pushing through the hollow needle (5) and a separating surface (48) forming an extension of the separating plane (16).

2. An ultrasonic Doppler probe according to claim 1, characterised in that in the case of a spring clip opened by pressure closing the clip cheeks (18, 18') are forced apart, so that the probe head (11) and needle guide (17) at the separating plane (16) and the coupling part (41) at the separating surface (48) are opened in lateral wedge-like manner so that the ultrasonic Doppler probe (1) with coupling part (41) can be extended sideways from the hollow needle (5).

3. An ultrasonic Doppler probe according to claim 1, characterised in that the replaceable coupling part (41) has an inclined contact surface (46) for the transmission of sound waves to the body to be examined.

4. An ultrasonic Doppler probe according to claim 3, characterised in that the probe head (11) has a planar contact surface (19) for the transmission of sound waves via the coupling part (41) into a body to be examined.

5. An ultrasonic Doppler probe according to claim 2, characterised in that the probe head (11) has a two-stage contact surface (19) for transmitting sound waves via the coupling part (41) into a body to be examined.

## Revendications

1. Sonde aux ultrasons à effet Doppler (1) pour localiser des vaisseaux sanguins dans un corps humain et les piquer avec une canule (5), la sonde aux ultrasons à effet Doppler (1) étant constituée :
d'une tête de sonde (11), disposée dans un support (14) et qui présente un émetteur d'ultrasons (11') et un récepteur d'ultrasons (11''),
d'un guide d'aiguille (17) disposé sous forme de perçage dans la tête de sonde (11), entouré au moins partiellement par l'émetteur d'ultrasons (11') et le récepteur d'ultrasons (11'') et destiné à introduire et faire passer la canule (5), et d'un élément d'accouplement remplaçable (41) pourvu d'un remplissage (43) réalisé dans un matériau présentant des propriétés ultrasonores approximativement identiques à celles du corps à analyser,
le support (14) étant une pince à ressort avec deux moitiés de pince (14', 14'') reliées par une articulation pivotante (15) et pourvues de joues de serrage (18, 18') appliquées l'une contre l'autre et définissant un plan de séparation (16), et la tête de sonde (11) étant disposée dans les joues de serrage (18, 18') de telle sorte que l'émetteur d'ultrasons (11') et le récepteur d'ultrasons (11'') se trouvent dans les deux moitiés de pince (14', 14'') en étant séparés par le plan de séparation (16),
**caractérisée** en ce que la tête de sonde (11) est réalisée cylindrique, en ce que le perçage est disposé coaxialement à la tête de sonde (11), en ce que l'émetteur d'ultrasons (11') et le récepteur d'ultrasons (11'') dépassent hors du support (14) dans la direction de piquage de l'aiguille, et en ce que l'élément d'accouplement (41) est une pièce moulée cylindrique (42) qui entoure partiellement la tête de sonde (11) et présente un perçage longitudinal débouchant (47) pour faire passer la canule (5), et une face de séparation (48) qui constitue un prolongement du plan de séparation (16).

2. Sonde aux ultrasons à effet Doppler selon la revendication 1, **caractérisée** en ce que, lors de l'ouverture de la pince à ressort en la pressant, les joues de serrage (18, 18') sont écartées, de sorte que la tête de sonde (11) et le guide d'aiguille (17) sur le plan de séparation (16), et l'élément d'accouplement (41) sur la face de séparation (48), sont ouverts latéralement en forme de coin, par où la sonde aux ultrasons à effet Doppler (1) peut être, conjointement avec l'élément d'accouplement (41), sortie latéralement de la canule (5).

3. Sonde aux ultrasons à effet Doppler selon la revendication 1, **caractérisée** en ce que l'élément d'accouplement remplaçable (41) présente une face de contact inclinée (46) pour la transmission des ondes sonores sur le corps à analyser.

4. Sonde aux ultrasons à effet Doppler selon la revendication 3, **caractérisée** en ce que la tête de sonde (11) présente une face de contact plane (19) pour la transmission des ondes sonores dans un corps à analyser par l'intermédiaire de l'élément d'accouplement (41).

5. Sonde aux ultrasons à effet Doppler selon la revendication 2, **caractérisée** en ce que la tête de sonde (11) présente une face de contact (19) à deux paliers pour la transmission des ondes sonores dans un corps à analyser par l'intermédiaire de l'élément d'accouplement (41).
